# EUROPEAN PATENT APPLICATION

(11) **EP 4 768 575 A1**
(43) Date of publication of application: **01.07.2026**
(21) Application number: 25782881.4
(22) Date of filing: 28.03.2025
(51) Int. Cl.: C12N 9/02, C12N 15/77, C12P 13/08, C12P 13/10, C12P 13/06

(54) **MICROORGANISM COMPRISING VARIANT OF NICOTINAMIDE NUCLEOTIDE TRANSHYDROGENASE AND METHOD FOR PRODUCING L-AMINO ACID USING SAME**

(30) Priority: 04.04.2024 KR 20240046185
(71) Applicant: CJ CHEILJEDANG CORPORATION, Seoul 04560 (KR)
(72) Inventor: EOM, Ga Eul, Seoul 04560 (KR); KIM, Kyungrim, Seoul 04560 (KR); JIN, Chorok, Seoul 04560 (KR); JANG, Yongjae, Seoul 04560 (KR); KIM, Ye-Eun, Seoul 04560 (KR); KIM, Ju Eun, Seoul 04560 (KR); KIM, Hyun Ah, Seoul 04560 (KR); SHIM, Jihyun, Seoul 04560 (KR); KIM, Moonjung, Seoul 04560 (KR); SEO, Chang Il, Seoul 04560 (KR)
(74) Representative: Meissner Bolte Partnerschaft mbB
(86) International application number: PCT/KR2025/004111
(87) International publication number: WO 2025/211658

(57) **Abstract**

Provided are a variant polypeptide of a nicotinamide nucleotide transhydrogenase beta subunit; a polynucleotide encoding the variant polypeptide; a microorganism comprising the same; and use of the microorganism in producing an L-amino acid.

## Description

### [Technical Field]

The present disclosure relates to a microorganism comprising any one or more selected from a variant polypeptide of a nicotinamide nucleotide transhydrogenase beta subunit and a polynucleotide encoding the variant polypeptide; a method of producing an L-amino acid or a derivative thereof, the method comprising the step of culturing the microorganism in a medium; a composition for producing an L-amino acid or a derivative thereof, the composition comprising any one or more selected from the microorganism, a culture of the microorganism, and a fermentation product of the microorganism; and use of the microorganism in producing an L-amino acid or a derivative thereof.

### [Background Art]

Coryneform microorganisms are Gram-positive microorganisms that are frequently used in industrial production of substances with various applications such as feeds, pharmaceuticals, and foods comprising L-amino acids and various nucleic acids. In recent years, diamine, keto-acid, etc. are also produced from coryneform microorganisms.

In order to produce useful products through microbial fermentation, a demand for an energy source or a reducing power is increased, along with strengthening the biosynthetic pathway of a target product in microorganisms. Among them, nicotinamide adenine dinucleotide phosphate (NADPH) is an essential element in providing a reducing power. The oxidized form NADP⁺ and the reduced form NADPH are *in vivo* electron transfer materials and are involved in various synthesis processes. Among the central metabolic pathways, NADPH is known to be mainly produced by 1) the oxidative pentose phosphate pathway and 2) the NADP-dependent isocitrate dehydrogenase (Icd gene) of the TCA pathway. In addition, various microorganisms have malate enzyme, glucose dehydrogenase, and non-phosphorylating glyceraldehyde-3-phosphate dehydrogenase in various alternative pathways to supply NADPH.

Further, regardless of the central metabolic pathway, NADPH-producing enzymes comprise transhydrogenase, Ferredoxin:NADP⁺ oxidoreductase, etc.

With the coexistence of the demand for various pathway enhancements and energy sources or reducing power, there is still a growing need for methods of increasing the production capacity of desired L-amino acids or derivatives thereof.

### [Prior Art Documents]

### [Patent Documents]

(Patent Document 1) 1. U.S. Patent No. 7,629,142 B2

### [Disclosure]

### [Technical Problem]

An object to be achieved by the present disclosure is to provide a microorganism comprising a variant of nicotinamide nucleotide transhydrogenase, and a method of producing an L-amino acid using the same.

### [Technical Solution]

One aspect of the present disclosure provides a variant polypeptide of a nicotinamide nucleotide transhydrogenase beta subunit, in which an amino acid corresponding to position 289 of SEQ ID NO: 11 is substituted with alanine; cysteine; isoleucine; methionine; or threonine.

In one specific embodiment, the variant polypeptide may consist of an amino acid sequence of SEQ ID NO: 74, SEQ ID NO: 76, SEQ ID NO: 78, SEQ ID NO: 80, or SEQ ID NO: 82.

In another specific embodiment, the nicotinamide nucleotide transhydrogenase beta subunit may be encoded by *pntB* gene.

Another aspect of the present disclosure provides a nicotinamide nucleotide transhydrogenase comprising the variant polypeptide.

Still another aspect of the present disclosure provides a polynucleotide encoding the variant polypeptide.

Still another aspect of the present disclosure provides a microorganism comprising any one or more selected from the variant polypeptide; the polynucleotide encoding the variant polypeptide; and a vector comprising the same.

With regard to the microorganism according to any one of the aforementioned specific embodiments, the microorganism may be a microorganism of the genus *Corynebacterium.*

With regard to the microorganism according to any one of the aforementioned specific embodiments, the microorganism of the genus *Corynebacterium* may be *Corynebacterium glutamicum.*

With regard to the microorganism according to any one of the aforementioned specific embodiments, the microorganism may have an increased ability to produce an L-amino acid or a derivative thereof, as compared to a microorganism comprising the polypeptide of SEQ ID NO: 11 or a polynucleotide encoding the same.

With regard to the microorganism according to any one of the aforementioned specific embodiments, the microorganism may have an increased ability to produce L-threonine, L-arginine, or L-homoserine.

Still another aspect of the present disclosure provides a method of producing an L-amino acid or a derivative thereof, the method comprising the step of culturing the microorganism in a medium.

In one specific embodiment, the method may further comprise the step of recovering the L-amino acid or the derivative thereof from the cultured microorganism; a culture of the microorganism; a fermentation product of the microorganism; or the culture medium.

In another specific embodiment, the method may be a method of producing L-threonine, L-arginine, or L-homoserine.

Still another aspect of the present disclosure provides a composition for producing an L-amino acid, the composition comprising any one or more selected from the variant polypeptide; the polynucleotide encoding the variant polypeptide; a vector comprising the same; a microorganism comprising the vector; a culture of the microorganism; and a fermentation product of the microorganism.

In one specific embodiment, the composition may be a composition for producing L-threonine, L-arginine, or L-homoserine.

### [Advantageous Effects]

When a microorganism comprising a variant polypeptide of a nicotinamide nucleotide transhydrogenase beta subunit of the present disclosure is cultured, it is possible to produce L-amino acids or derivatives thereof with high yields, as compared to a microorganism having the existing unmodified polypeptide.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a variant polypeptide of a nicotinamide nucleotide transhydrogenase beta subunit, in which an amino acid corresponding to position 289 of SEQ ID NO: 11 is substituted with alanine; cysteine; isoleucine; methionine; or threonine.

As used herein, the term "nicotinamide nucleotide transhydrogenase" may refer to an enzyme that maintains the redox balance of cells through the reaction [H⁺ + NADP⁺ + NADH ↔ H⁺ + NADPH + NAD⁺], which exists as a homodimer with each protomer comprising a proton-translocating transmembrane domain and two soluble nucleotide binding domains that mediate hydride transfer between NAD(H) and NADP(H). In the present disclosure, the nicotinamide nucleotide transhydrogenase may refer to a transmembrane pyridine nucleotide transhydrogenase that catalyzes the reduction of NADP⁺ to NADPH via the oxidation of NADH to NAD⁺, and is composed of two subunits, alpha and beta, encoded by *pntA* and *pntB* genes, respectively.

The nicotinamide nucleotide transhydrogenase of the present disclosure may be used interchangeably with pntAB. Specifically, the nicotinamide nucleotide transhydrogenase of the present disclosure may be a protein having the activity of nicotinamide nucleotide transhydrogenase encoded by *pntAB* (*pntA* and *pntB*) gene, but its type is not particularly limited, as long as it has the activity corresponding to nicotinamide nucleotide transhydrogenase.

The nicotinamide nucleotide transhydrogenase encoded by *pntAB* gene is known in the art, and the amino acid and polynucleotide sequences of the nicotinamide nucleotide transhydrogenase may be obtained from a known database (e.g., GenBank), but is not limited thereto.

For example, the nicotinamide nucleotide transhydrogenase may be composed of alpha subunit PntA and beta subunit PntB.

The nicotinamide nucleotide transhydrogenase may comprise an amino acid sequence of SEQ ID NO: 10 or an amino acid sequence having 60% or more homology or identity thereto; and an amino acid sequence of SEQ ID NO: 11 or an amino acid sequence having 60% or more homology or identity thereto, but is not limited thereto as long as it has the nicotinamide nucleotide transhydrogenase activity. Specifically, any protein having the amino acid sequences of SEQ ID NOS: 10 and 11, in which part of the sequences is deleted, modified, substituted, or added, may be comprised in the nicotinamide nucleotide transhydrogenase as long as it exhibits the efficacy corresponding to that of nicotinamide nucleotide transhydrogenase. Further, the protein may have or comprise amino acid sequences having at least 60%, 70%, 80%, 85%, 90%, 95%, 96%, 97%, 98%, or 99% or more homology or identity to the amino acid sequences of SEQ ID NOS: 10 and 11, may consist of the amino acid sequences, or may essentially consisting of the amino acid sequences.

The protein of the present disclosure, which is a subject of the introduction of the mutation, may be a protein having the nicotinamide nucleotide transhydrogenase activity. Specifically, the protein may consist of the amino acid sequence of SEQ ID NO: 11 or may comprise the same, but is not limited thereto. Addition of a meaningless sequence upstream or downstream of the amino acid sequence of SEQ ID NO: 11, a naturally occurring mutation, or a silent mutation thereof is not excluded, and as long as a protein has activity identical or corresponding to that of the protein comprising the amino acid sequence of SEQ ID NO: 11, it may belong to the protein which is a subject of the introduction of the mutation of the present disclosure. For example, the protein which is a subject of the introduction of the mutation of the present disclosure may be a protein consisting of an amino acid sequence having 60%, 70%, 75%, 80%, 85%, 90%, 95%, 96%, 97%, 98%, 99%, 99.5%, 99.7% or 99.9% or more homology or identity to the amino acid sequence of SEQ ID NO: 11. Further, it is obvious that any polypeptide having an amino acid sequence with deletion, modification, substitution, conservative substitution, or addition in part of the sequence may also fall within the scope of the polypeptide which is a subject of the introduction of the mutation of the present disclosure, as long as it is an amino acid sequence having such a homology or identity and exhibiting efficacy corresponding to that of the nicotinamide nucleotide transhydrogenase beta subunit polypeptide.

As used herein, the term "variant polypeptide", "variant protein" or "variant" refers to a polypeptide in which one or more amino acids are conservatively substituted and/or modified so as to be different from the amino acid sequence of the variant before modification, while retaining functions or properties. Such a variant may generally be identified by modifying one or more amino acids in the amino acid sequence of the polypeptide and evaluating the properties of the modified polypeptide. In other words, the ability of the variant may be increased, unchanged, or decreased, as compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "variant" may also be used interchangeably with modified polypeptide, modified protein, mutant, mutein, etc., and any term is not limited thereto, as long as it is used in a sense of being mutated.

The variant may also comprise deletion or addition of amino acids that have minimal influence on the properties and secondary structure of the polypeptide. For example, the polypeptide may be conjugated with an N-terminal signal (or leader) sequence of a protein involved in the transfer of proteins co-translationally or post-translationally. Further, the polypeptides may also be conjugated with another sequence or linker to identify, purify, or synthesize the polypeptides.

In the variant polypeptide of the present disclosure, the amino acid corresponding to position 289 from the N-terminus of SEQ ID NO: 11 may be substituted with another amino acid.

The "variant polypeptide" may be referred to as "nicotinamide nucleotide transhydrogenase beta subunit variant polypeptide", "nicotinamide nucleotide transhydrogenase beta subunit variant", "variant pntB", "pntB variant", etc. The nicotinamide nucleotide transhydrogenase beta subunit polypeptide of the present disclosure, which is a subject of the introduction of the mutation, may be used interchangeably with "PntB", and may be encoded by *pntB* gene without particular limitation, and may be encoded by *pntB* gene derived from *Corynebacterium variabile,* but is not limited thereto.

In the amino acid sequence before modification of the nicotinamide nucleotide transhydrogenase beta subunit that is the subject of the mutation of the present disclosure, i.e., the sequence to be a parent sequence, the amino acid before modification corresponding to position 289 of SEQ ID NO: 11 may be, but is not limited to, serine (S).

With regard to the variant polypeptide of the nicotinamide nucleotide transhydrogenase beta subunit of the present disclosure, the amino acid corresponding to position 289 of the amino acid sequence of SEQ ID NO: 11 may be substituted with an amino acid which is different from the amino acid before substitution.

In one specific embodiment, with regard to the variant polypeptide, the amino acid corresponding to position 289 of the amino acid sequence of SEQ ID NO: 11 may be substituted with another amino acid than serine, which is the amino acid before substitution.

In one specific embodiment, with regard to the variant polypeptide, the amino acid corresponding to position 289 of SEQ ID NO: 11 may be substituted with an amino acid selected from the group consisting of asparagine, valine, glycine, leucine, arginine, alanine, methionine, threonine, glutamine, proline, isoleucine, tryptophan, phenylalanine, histidine, cysteine, tyrosine, lysine, aspartate, and glutamic acid.

In any one embodiment of the aforementioned embodiments, with regard to the variant polypeptide provided in the present disclosure, the amino acid corresponding to position 289 from the N-terminus of SEQ ID NO: 11 may be substituted with alanine; cysteine; isoleucine; methionine; or threonine.

In another embodiment of the aforementioned embodiments, the variant polypeptide provided in the present disclosure may comprise an amino acid sequence having at least 60%, 70%, 75%, 80%, 85%, 90%, 95%, 96%, 97%, 98%, 99%, 99.5%, or 99.8% or more, or less than 100% homology or identity to SEQ ID NO: 11.

For example, the variant polypeptide of the present disclosure may comprise an amino acid sequence having at least 60%, 70%, 75%, 80%, 85%, 90%, 95%, 96%, 97%, 98%, 99%, 99.5%, or 99.8% or more homology or identity to SEQ ID NO: 11 while the amino acid corresponding to position 289 of the amino acid sequence represented by SEQ ID NO: 11 is fixed to alanine; cysteine; isoleucine; methionine; or threonine. Further, it is apparent that any variant polypeptide having an amino acid sequence with deletion, modification, substitution, conservative substitution, or addition in part of the sequence may also fall within the scope of the present disclosure, as long as it is an amino acid sequence having such a homology or identity and exhibiting efficacy corresponding to that of the variant polypeptide of the present disclosure.

Meanwhile, those skilled in the art may identify an amino acid which corresponds to position 289 of the amino acid sequence of SEQ ID NO: 11 of the present disclosure, in an arbitrary amino acid sequence through sequence alignment known in the art. Unless otherwise described in the present disclosure, when "an amino acid at a specific position of a specific sequence number" is described, it is apparent that the amino acid also comprises "an amino acid at the corresponding position" in an arbitrary amino acid sequence.

In still another specific embodiment, the variant polypeptide may consist of an amino acid sequence of SEQ ID NO: 74, SEQ ID NO: 76, SEQ ID NO: 78, SEQ ID NO: 80, or SEQ ID NO: 82.

Specifically, the variant polypeptide of the present disclosure may have or comprise the amino acid sequence of SEQ ID NO: 74, SEQ ID NO: 76, SEQ ID NO: 78, SEQ ID NO: 80, or SEQ ID NO: 82, or an amino acid sequence having at least 60%, 70%, 80%, 85%, 90%, 95%, 96%, 97%, 98%, or 99% or more homology or identity to the amino acid sequence of SEQ ID NO: 74, 76, 78, 80, or 82, or may consist of the amino acid sequence, or may essentially consist of the amino acid sequence.

Further, the variant polypeptide of the present disclosure may also comprise a polypeptide having additions or deletions of sequences, which do not alter the function of the variant polypeptide, at the N-terminus, C-terminus, and/or inside of the amino acid sequence of the variant polypeptide of the present disclosure, a naturally occurring mutation, a silent mutation, or a conservative substitution.

The "different amino acid" is not limited as long as it differs from the amino acid before substitution. Meanwhile, in the present disclosure, when expressed as 'a specific amino acid is substituted', it is obvious that the amino acid is substituted with an amino acid different from the amino acid before substitution, even though it is not separately indicated that the amino acid is substituted with a different amino acid.

The amino acid may be generally classified, based on similarity in polarity, electric charge, solubility, hydrophobicity, hydrophilicity, and/or amphipathic nature of residues.
Examples of this classification may comprise positively charged (basic) amino acids such as arginine, lysine, and histidine; negatively charged (acidic) amino acids such as glutamic acid and aspartic acid; amino acids with nonpolar side chains (nonpolar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, and tyrosine. However, they are not necessarily limited thereto.

For example, when it is described that "the amino acid corresponding to position 289 of SEQ ID NO: 11 is substituted with a different amino acid", it may mean that the amino acid is substituted with asparagine, valine, glycine, alanine, glutamic acid (glutamate), phenylalanine, arginine, aspartate, cysteine, glutamine, histidine, proline, isoleucine, tyrosine, lysine, tryptophan, methionine, threonine, or leucine, excluding serine.

As used herein, although the expression "protein having an amino acid sequence described by a specific sequence number" is used, it is obvious that any protein having an amino acid sequence comprising deletion, modification, substitution, conservative substitution, or addition of some sequence may also be used in the present disclosure as long as the protein has activity identical or equivalent to the protein consisting of the amino acid sequence of the corresponding sequence number. Examples thereof do not exclude addition of a sequence, which does not alter the function of the protein, upstream or downstream of the amino acid sequence, a mutation that may occur naturally, a silent mutation thereof, or a conservative substitution, as long as it has activity identical or corresponding to that of the variant protein, and it would be obvious that even a protein with such a sequence addition or mutation falls within the scope of the present disclosure.

The "position N" of the present disclosure may comprise the position N and an amino acid position corresponding to the position N. Specifically, the position N may comprise an amino acid position corresponding to an arbitrary amino acid residue in a mature polypeptide, disclosed in a specific amino acid sequence. The specific amino acid sequence may be the amino acid sequence of SEQ ID NO: 11.

As used herein, the term "corresponding to" refers to an amino acid residue at a position listed in a polypeptide, or an amino acid residue similar to, identical to, or homologous to a residue listed in a polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 11, and based on this, each amino acid residue of the amino acid sequence may be numbered with reference to the numerical position of the amino acid residue corresponding to the amino acid residue of SEQ ID NO: 11. For example, a sequence alignment algorithm as described in the present disclosure may determine the position of an amino acid, or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto, and a sequence alignment program, a pairwise sequence comparison algorithm, etc., which known in the art, may be appropriately used.

As used herein, the term "conservative substitution" means substitution of one amino acid with another amino acid having similar structural and/or chemical properties. Such an amino acid substitution may generally occur based on similarity in polarity, charge, solubility, hydrophobicity, hydrophilicity, and/or amphipathic nature of the residues. For example, positively charged (basic) amino acids comprise arginine, lysine, and histidine; negatively charged (acidic) amino acids comprise glutamic acid and aspartate; aromatic amino acids comprise phenylalanine, tryptophan, and tyrosine; and hydrophobic amino acids comprise alanine, valine, isoleucine, leucine, methionine, phenylalanine, tyrosine, and tryptophan. Further, amino acids may also be classified into amino acids with electrically charged side chains and amino acids with uncharged side chains. The amino acids with electrically charged side chains comprise aspartic acid, glutamic acid, lysine, arginine, and histidine, and the amino acids with uncharged side chains may be classified again into nonpolar amino acids and polar amino acids. The nonpolar amino acids comprise glycine, alanine, valine, leucine, isoleucine, methionine, phenylalanine, tryptophan, and proline, and the polar amino acids comprise serine, threonine, cysteine, tyrosine, asparagine, and glutamine. Usually, conservative substitution may hardly affect or not affect the activity of produced polypeptides. Usually, conservative substitution may hardly affect or not affect the activity of proteins or polypeptides.

Still another aspect of the present disclosure provides a nicotinamide nucleotide transhydrogenase comprising the variant polypeptide.

The nicotinamide nucleotide transhydrogenase may comprise PntA; and the variant polypeptide PntB.

In other words, the nicotinamide nucleotide transhydrogenase of the present disclosure may be composed of a nicotinamide nucleotide transhydrogenase alpha subunit and the variant polypeptide of the nicotinamide nucleotide transhydrogenase beta subunit of the present disclosure.

Still another aspect of the present disclosure provides a polynucleotide encoding the variant polypeptide; or a polynucleotide encoding the nicotinamide nucleotide transhydrogenase comprising the variant polypeptide.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant polypeptide.

The polynucleotide encoding the variant polypeptide of the nicotinamide nucleotide transhydrogenase beta subunit of the present disclosure may comprise any polynucleotide sequence without limitation as long as it is a polynucleotide encoding the variant polypeptide capable of forming nicotinamide nucleotide transhydrogenase, together with the alpha subunit.

For example, the polynucleotide encoding the variant polypeptide of the nicotinamide nucleotide transhydrogenase beta subunit of the present disclosure may be a polynucleotide sequence encoding an amino acid sequence, in which the amino acid corresponding to position 289 of SEQ ID NO: 11 is substituted with alanine; cysteine; isoleucine; methionine; or threonine, but is not limited thereto.

For example, the polynucleotide of the present disclosure may comprise a nucleotide sequence encoding the amino acid sequence of SEQ ID NO: 74, SEQ ID NO: 76, SEQ ID NO: 78, SEQ ID NO: 80, or SEQ ID NO: 82. For example, the polynucleotide of the present disclosure may have or comprise a sequence of SEQ ID NO: 75, SEQ ID NO: 77, SEQ ID NO: 79, SEQ ID NO: 81, or SEQ ID NO: 83. Further, the polynucleotide of the present disclosure may consist of or essentially consist of the sequence of SEQ ID NO: 75, SEQ ID NO: 77, SEQ ID NO: 79, SEQ ID NO: 81, or SEQ ID NO: 83.

The polynucleotide of the present disclosure may undergo various modifications in the coding region within the scope that does not change the amino acid sequence of the variant polypeptide of the present disclosure in consideration of codon degeneracy or codons preferred in organisms that are intended to express the variant polypeptide of the present disclosure. Therefore, it is obvious that, due to codon degeneracy, a polynucleotide to be translated into a polypeptide consisting of the amino acid sequence of the variant polypeptide of the present disclosure or a polypeptide having homology or identity thereto may also be comprised. For example, the polynucleotide of the present disclosure may consist of or may comprise the sequence of SEQ ID NO: 75, SEQ ID NO: 77, SEQ ID NO: 79, SEQ ID NO: 81 or SEQ ID NO: 83; or a degenerated sequence thereof.

For example, the polynucleotide of the present disclosure may comprise substitution of codons corresponding to codons at positions 865 to 867 of SEQ ID NO: 13 with a codon encoding a different amino acid, e.g., an amino acid selected from alanine, cysteine, isoleucine, methionine, or threonine in a nucleotide sequence having 60%, 70%, 75%, 80%, 85%, 90%, 95%, 96%, 97%, 98%, 99%, 99.5%, 99.7% or 99.8% or more homology or identity to the sequence of SEQ ID NO: 13, but is not limited thereto. It is also apparent that a variant having a polynucleotide sequence having deletion, modification, substitution, conservative substitution, or addition of some sequence also falls within the scope of the present disclosure as long as the polynucleotide sequence has such a homology or identity and encodes the amino acid sequence of the variant polypeptide of the nicotinamide nucleotide transhydrogenase beta subunit of the present disclosure.

For another example, the polynucleotide of the present disclosure may have or comprise a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 75, SEQ ID NO: 77, SEQ ID NO: 79, SEQ ID NO: 81, or SEQ ID NO: 83, or may consist of or may essentially consist of a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 75, SEQ ID NO: 77, SEQ ID NO: 79, SEQ ID NO: 81, or SEQ ID NO: 83.

Further, the polynucleotide of the present disclosure may comprise a probe that may be prepared from a known gene sequence, for example, and may comprise any sequence without limitation as long as it is a sequence that is able to hybridize with a complementary sequence to the entirety or a part of the polynucleotide sequence of the present disclosure under stringent conditions.

The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof comprise a condition in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "homology" or "identity" means the relevance between two given amino acid sequences or nucleotide sequences and may be expressed as a percentage. The terms "homology and identity" may often be used interchangeably.

The sequence homology or identity of conserved polynucleotides or polypeptides is determined by standard alignment algorithms, and a default gap penalty established by the program being used may be used together. Substantially, homologous or identical sequences may generally hybridize with the entirety or a part of the sequence under moderately or highly stringent conditions. It is apparent that hybridization also comprises hybridization of a polynucleotide with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387(1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST or ClustalW of the National Center for Biotechnology Information may be used to determine the homology, similarity, or identity.

The homology, similarity, or identity of polynucleotides or polypeptides may be, for example, determined by comparing sequence information using, for example, the GAP computer program, such as Needleman et al.(1970), J Mol Biol. 48:443, as disclosed in Smith and Waterman, Adv. Appl. Math(1981) 2:482. Briefly, the GAP program defines the homology, similarity, or identity as the value obtained by dividing the number of similarly aligned symbols (i.e., nucleotides or amino acids) by the total number of symbols in the shorter of the two sequences. Default parameters for the GAP program may comprise (1) a binary comparison matrix (comprising a value of 1 for identities and 0 for non-identities) and the weighted comparison matrix of Gribskov et al. (1986) Nucl. Acids Res. 14: 6745, as disclosed in Schwartz and Dayhoff, eds., Atlas Of Protein Sequence And Structure, National Biomedical Research Foundation, pp. 353-358(1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" used herein represents the relevance between sequences.

Still another aspect of the present disclosure provides a vector comprising the polynucleotide of the present disclosure. The vector may be an expression vector for expressing the polynucleotide in a microorganism, but is not limited thereto.

As used herein, the term "vector" may comprise a DNA construct comprising a nucleotide sequence of a polynucleotide encoding a polypeptide of interest which is operably linked to a suitable expression regulatory region (or expression control sequence) so that the polypeptide of interest may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. Once transformed into a suitable microorganism, the vector may replicate or function independently from the host genome, or may integrate into the genome thereof.

The vector that may be used in the present disclosure is not particularly limited, and any vector known in the art may be used. Examples of the vectors commonly used may comprise natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, etc. may be used, and as a plasmid vector, those based on pDZ, pBR, pUC, pBluescriptll, pGEM, pTZ, pCL and pET, etc. may be used. Specifically, pDZ, pDC, pDCM2, pDC24, pACYC177, pACYC184, pCL, pECCG117, pUC19, pBR322, pMW118, pCC1BAC vectors, etc. may be used.

For example, the polynucleotide encoding the target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cytotoxic agents, or expression of surface polypeptides, may be used. Only cells expressing the selection marker are able to survive or show different phenotypes under the environment treated with the selective agent, and thus the transformed cells may be selected.

As used herein, the term "transformation" means that a vector comprising a polynucleotide encoding a target polypeptide is introduced into a microorganism or a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. Further, the polynucleotide comprises DNA and/or RNA encoding the target polypeptide. The polynucleotide may be introduced in any form as long as it may be introduced into a microorganism and expressed. For example, the polynucleotide may be introduced into a microorganism in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

Further, the term "operably linked" means that the polynucleotide sequence is functionally linked to a promoter sequence that initiates and mediates transcription of the polynucleotide encoding the target variant polypeptide of the present disclosure.

The method of transforming the vector of the present disclosure comprises any method of introducing the nucleic acid into a cell, and may be performed by selecting a suitable standard technique known in the art according to the host cell. For example, the transformation method may comprise electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, etc., but is not limited thereto.

Still another aspect of the present disclosure provides a microorganism comprising any one or more selected from the variant polypeptide; the polynucleotide encoding the variant polypeptide; and a vector comprising the same.

Still another aspect of the present disclosure provides a microorganism further comprising any one or more selected from the nicotinamide nucleotide transhydrogenase alpha subunit; a polynucleotide encoding the alpha subunit; and a vector comprising the same.

In one specific embodiment, the microorganism of the present disclosure may be a microorganism having an ability to produce an L-amino acid or a derivative thereof.

As used herein, the term "microorganism (or strain)" comprises all wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism comprising genetic modification for production of a target polypeptide, protein, or product. As used herein, the terms "microorganism", "strain", and "microorganism" have the same meaning and may be used interchangeably without limitation.

As used herein, the term "recombinant microorganism" refers to a microorganism that has been genetically modified and exhibits a different genotype and/or phenotype, compared to a naturally occurring microorganism (e.g., when the genetic modifications have an effect on a coding nucleic acid sequence of a microorganism), and may comprise progeny or all potential progeny of the microorganism. As used herein, the terms "recombinant microorganism", "genetically modified microorganism", "recombinant host cell", "recombinant cell", and "recombinant strain" may be used interchangeably. The recombinant microorganism, for example, may express genes that are not found in the native (non-recombinant) form; or may not express genes that are expressed in its native form, or may express native genes in a manner different from that expressed in its native form.

As used herein, the term "microorganism producing an L-amino acid or a derivative thereof" refers to a prokaryotic or eukaryotic microbial strain capable of producing the L-amino acid or derivative thereof within a living organism, and may comprise both a microorganism prepared by providing the ability to produce the L-amino acid or derivative thereof to a parent strain without the ability to produce the L-amino acid or derivative thereof, or a microorganism that inherently has the ability to produce the L-amino acid or derivative thereof. The ability to produce the L-amino acid or derivative thereof may be conferred or enhanced by species improvement.

In one embodiment, the microorganism of the present disclosure may be a microorganism having naturally the variant polypeptide of the nicotinamide nucleotide transhydrogenase beta subunit or the ability to produce the L-amino acid or derivative thereof; or a microorganism in which the variant polypeptide of the present disclosure or the polynucleotide encoding the same (or the vector comprising the polynucleotide) is introduced and/or the ability to produce the L-amino acid or derivative thereof is provided to a parent strain without the variant polypeptide of the nicotinamide nucleotide transhydrogenase beta subunit or the ability to produce the L-amino acid or derivative thereof, but is not limited thereto.

In one embodiment, the microorganism of the present disclosure may comprise any of a microorganism comprising the variant polypeptide sequence of the nicotinamide nucleotide transhydrogenase beta subunit of the present disclosure due to mutation of the gene encoding the variant polypeptide of the nicotinamide nucleotide transhydrogenase beta subunit on the chromosome, and/or a microorganism comprising the variant polypeptide of the nicotinamide nucleotide transhydrogenase beta subunit of the present disclosure by introducing the polynucleotide encoding the variant polypeptide of the nicotinamide nucleotide transhydrogenase beta subunit of the present disclosure or the vector comprising the same, but is not limited thereto.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. For example, the unmodified microorganism may be a strain into which the variant polypeptide of the nicotinamide nucleotide transhydrogenase beta subunit described herein is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

The microorganism having the ability to produce an L-amino acid or a derivative thereof of the present disclosure may be a microorganism comprising any one or more of the variant polypeptide of the present disclosure, the polynucleotide of the present disclosure, and the vector comprising the polynucleotide of the present disclosure; a microorganism which is modified to express the variant polypeptide of the present disclosure or the polynucleotide of the present disclosure; a microorganism (e.g., recombinant strain) expressing the variant polypeptide of the present disclosure or the polynucleotide of the present disclosure; or a microorganism (e.g., recombinant strain) having the activity of the variant polypeptide of the present disclosure, but is not limited thereto.

For example, the microorganism of the present disclosure is a cell or microorganism which is transformed with the polynucleotide encoding the variant polypeptide of the present disclosure or the vector comprising the same to express the variant polypeptide of the present disclosure, and the strain of the present disclosure may comprise any microorganism that is able to produce the L-amino acid or derivative thereof by comprising the variant polypeptide of the present disclosure. For example, the microorganism of the present disclosure may be a recombinant strain having the increased ability to produce the L-amino acid or derivative thereof due to expression of the variant polypeptide of the nicotinamide nucleotide transhydrogenase beta subunit or expression of nicotinamide nucleotide transhydrogenase comprising the variant polypeptide of the present disclosure by introducing the polynucleotide encoding the variant polypeptide of the present disclosure into a natural wild-type microorganism or a microorganism having the ability to produce the L-amino acid or derivative thereof. The recombinant strain having the increased ability to produce the L-amino acid or derivative thereof may be a microorganism in which the ability to produce the L-amino acid or derivative thereof is increased, as compared to the natural wild-type microorganism or unmodified microorganism (e.g., a microorganism expressing the wild-type nicotinamide nucleotide transhydrogenase or a microorganism not expressing the variant polypeptide of the present disclosure), but is not limited thereto. For example, the microorganism having the increased ability to produce the L-amino acid or derivative thereof of the present disclosure may be a microorganism in which the ability to produce the L-amino acid or derivative thereof is increased, as compared to the microorganism comprising the polypeptide of SEQ ID NO: 11 or the polynucleotide encoding the same, but is not limited thereto. For example, the unmodified microorganism, which is the target strain being compared to determine whether or not the ability to produce the L-amino acid or derivative thereof is increased, may be a wild-type *Corynebacterium glutamicum* strain, KCCM 12502P (CA09-0903); CJR2 or KCCM12120P, but is not limited thereto.

The microorganism of the present disclosure may comprise any microorganism which is able to express the variant polypeptide of the nicotinamide nucleotide transhydrogenase beta subunit of the present disclosure by a variety of known methods in addition to introduction of the nucleotide or vector.

For example, the microorganism having the increased ability to produce the L-amino acid or derivative thereof may have an increased ability to produce the L-amino acid or derivative thereof of about 1% or more, specifically, about 1% or more, about 2% or more, about 3% or more, about 4% or more, about 5% or more, about 6% or more, about 7% or more, about 8% or more, about 9% or more, about 10% or more, about 11% or more, about 12% or more, about 13% or more, about 14% or more, about 15% or more, about 16% or more, about 17% or more, about 18% or more, about 19% or more, about 20% or more, about 25% or more, about 30% or more, about 35% or more, about 40% or more, about 50% or more (the upper limit is not particularly limited, but may be, for example, about 200% or less, about 150% or less, about 100% or less, about 90% or less, about 80% or less, about 70% or less, about 60% or less, about 55% or less, or about 50% or less), as compared to that of the parent strain before modification or unmodified microorganism, but the increased amount is not limited thereto as long as the production ability has an increased amount of a + value, as compared to the ability to the L-amino acid or derivative thereof of the parent strain before modification or the unmodified microorganism. In another example, the recombinant strain having the increased ability to produce the L-amino acid or derivative thereof may have an increased ability to produce the L-amino acid or derivative thereof of about 1.01 times or more, about 1.02 times or more, about 1.03 times or more, about 1.04 times or more, 1.05 times or more, 1.06 times or more, 1.07 times or more, 1.08 times or more, 1.09 times or more, about 1.1 times or more, about 1.11 times or more, about 1.12 times or more, about 1.13 times or more, about 1.14 times or more, about 1.15 times or more, about 1.16 times or more, about 1.17 times or more, about 1.18 times or more, about 1.19 times or more, about 1.20 times or more, about 1.25 times or more, about 1.30 times or more, about 1.35 times or more, about 1.40 times or more, about 1.45 times or more, or about 1.50 times or more (the upper limit is not particularly limited, but may be, for example, about 10 times or less, about 5 times or less, about 3 times or less, about 2 times or less, or about 1.5 times or less), as compared to that of the parent strain before modification or the unmodified microorganism, but is not limited thereto. As used herein, the term "about" refers to a range which comprises all of ±0.5, ±0.4, ±0.3, ±0.2, ±0.1, etc., and comprises all of the values that are equivalent or similar to those following the term "about", but the range is not limited thereto.

With regard to the microorganism according to any one of the aforementioned specific embodiments, the microorganism of the present disclosure may be a microorganism of the genus *Corynebacterium* (*Corynebacteria* sp.), the genus *Escherichia* (*Escherichia* sp.), the genus *Erwinia* (Erwinia sp.), the genus *Serratia* (*Serratia* sp.), the genus *Providencia* (*Providencia* sp.), the genus *Pseudomonas* (*Pseudomonas* sp.), the genus *Leptospira* (*Leptospira* sp.), the genus *Salmonella* (*Salmonella* sp.), the genus *Brevibacteria* (*Brevibacteria* sp.), the genus *Hypomononas* (*Hypomononas* sp.), the genus *Chromobacterium* (*Chromobacterium* sp.) and the genus *Norcardia* (*Norcardia* sp.), fungi, or yeasts, specifically, a microorganism of the genus *Corynebacterium,* but is not limited thereto.

For example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans*, *Corynebacterium testudinoris*, or *Corynebacterium flavescens.* Specifically, the microorganism of the present disclosure may be a microorganism of the genus *Corynebacterium,* more specifically, *Corynebacterium glutamicum,* but is not limited thereto.

Specifically, the microorganism of the present disclosure may be a microorganism of the genus *Corynebacterium,* more specifically, *Corynebacterium glutamicum*, but is not limited thereto.

Meanwhile, the microorganism of the genus *Corynebacterium* of the present disclosure, in which the ability to produce the L-amino acid or derivative thereof is improved, may comprise any of a natural wild-type microorganism itself, a microorganism of the genus *Corynebacterium* which has the improved ability to produce the L-amino acid or derivative thereof by increasing or decreasing the activity of a gene related to the production mechanism of the L-amino acid or derivative thereof, and a microorganism of the genus *Corynebacterium* which has the improved ability to produce the L-amino acid or derivative thereof by introducing or increasing the activity of a foreign gene. In one specific embodiment, the microorganism may comprise a microorganism of the genus *Corynebacterium,* in which the ability to produce the L-amino acid or derivative thereof is improved by introducing the variant polypeptide of the present disclosure or the nicotinamide nucleotide transhydrogenase comprising the same, but is not limited thereto.

As used herein, the term "increase" of the protein (polypeptide) activity means that the activity of the protein (polypeptide) is increased in host cells (microorganisms), as compared to the endogenous activity thereof. The increase may be used interchangeably with terms such as activation, up-regulation, overexpression, and enhancement, etc. The host cells (microorganisms) may be prokaryotic or eukaryotic microorganisms.

The increase of the protein (polypeptide) activity may comprise both exhibiting the protein (polypeptide) activity that was not originally possessed by host cells (microorganisms) and exhibiting improved protein (polypeptide) activity as compared to the endogenous activity or activity before modification.

For example, the "exhibiting the protein (polypeptide) activity that was not originally possessed" or "exhibiting improved protein (polypeptide) activity" may be caused by "introducing a protein (polypeptide)," but is not limited thereto.

As used herein, the term "introducing a protein (polypeptide)" means that a gene not originally possessed by a microorganism is expressed within the microorganism, thereby exhibiting the activity of the specific protein, or exhibiting the enhanced, increased, or improved polypeptide activity, as compared to the endogenous activity or activity before modification of the corresponding protein. For example, it may be caused by introducing a gene encoding the protein (polypeptide) into the host cells (microorganisms). For example, a polynucleotide encoding a specific protein (polypeptide) may be introduced into a chromosome within host cells (microorganisms), or a vector comprising the polynucleotide encoding the specific protein (polypeptide) may be introduced into the host cells (microorganisms), thereby exhibiting or improving activity thereof.

The "endogenous activity" means the activity of a specific protein (polypeptide) originally possessed by a host cell (microorganism) before the trait is changed or by an unmodified host cell (microorganism), when the trait is changed by genetic variation due to natural or artificial factors. This may be used interchangeably with "activity before modification".

The fact that the activity of the protein (polypeptide) is increased as compared to the endogenous activity means that the activity and/or concentration (expression level) of the protein (polypeptide) of the host cell (microorganism) is improved as compared to the activity and/or concentration (expression level) of the protein (polypeptide) originally possessed by the host cell (microorganism) before the trait is changed or by the unmodified host cell (microorganism).

For example, the increase indicates that the activity of the corresponding protein (polypeptide) originally absent appears, or its activity or concentration is generally increased to about 1% or more, about 10% or more, about 25% or more, about 50% or more, about 75% or more, about 100% or more, about 150% or more, about 200% or more, about 300% or more, about 400% or more, or about 500% or more, up to about 1000% or about 2000% or more, based on the activity or concentration in the host cell (microorganism) before the trait is changed or in the unmodified host cell (microorganism), but is not limited thereto.

The increase of the activity of the protein (polypeptide) may be achieved through the introduction of a foreign protein (polypeptide) or the increase of the activity of the endogenous protein (polypeptide). The increase of the activity of the protein (polypeptide) may be confirmed by enhancement in the degree of activity and the expression level of the corresponding protein (polypeptide) or by an increase in the amount of the product resulting from the activity of the corresponding protein (polypeptide).

The increase of the activity of the protein (polypeptide) may be achieved by various methods well known in the art, and the method is not limited as long as the activity of the target protein (polypeptide) may be increased as compared to that of the host cell (microorganism) before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the protein (polypeptide) of the present disclosure may be:
1) increase in the intracellular copy number of the polynucleotide encoding the protein (polypeptide);
2) modification of a gene expression regulatory region on a chromosome encoding the protein (polypeptide) (e.g., introduction of variations into the expression regulatory region, replacement with a sequence having stronger activity, or insertion of a sequence having stronger activity);
3) modification of a nucleotide sequence encoding an initiation codon or 5'-UTR region of the gene transcript encoding the protein (polypeptide);
4) modification of the amino acid sequence of the protein (polypeptide) to increase the activity of the protein (polypeptide);
5) modification of the polynucleotide sequence encoding the protein (polypeptide) to increase the activity of the protein (polypeptide) (e.g., modification of the polynucleotide sequence of the protein (polypeptide) coding gene to encode the protein (polypeptide) that has been modified to increase the activity of the protein (polypeptide));
6) introduction of a foreign protein (polypeptide) exhibiting the activity of the protein (polypeptide) or a foreign polynucleotide encoding the same;
7) codon optimization of the polynucleotide encoding the protein (polypeptide);
8) analysis of the tertiary structure of the protein (polypeptide) to select and modify or chemically modify the exposed site;
9) control of intracellular localization of the protein (polypeptide) or
10) a combination of two or more selected from 1) to 9), but is not particularly limited thereto.

For example,
1) the increase in the intracellular copy number of the polynucleotide encoding the protein (polypeptide) may be achieved by the introduction, into a host cell (microorganism), of a vector comprising the polynucleotide encoding the protein (polypeptide), which is operably linked to an appropriate regulatory sequence. Alternatively, the increase may be achieved by introducing one copy or two or more copies of the polynucleotide encoding the protein (polypeptide), which is operably linked to an appropriate regulatory sequence, into a chromosome of a host cell (microorganism). The introduction into the chromosome may be performed by the introduction, into the host cell (microorganism), of a vector capable of inserting the polynucleotide into the chromosome of the host cell (microorganism), but is not limited thereto. The vector is as described above. The regulatory sequence may be a native (derived from the same origin) or foreign (derived from different genes) sequence to the coding polynucleotide sequence, or a mutant sequence or other artificial sequence thereof, and may induce expression of the polynucleotide in the host cell (microorganism).
2) The replacement of a gene expression regulatory region (or expression regulatory sequence) on the chromosome encoding the protein (polypeptide) with a sequence with strong activity may be, for example, introduction of a mutation on the sequence through deletion, insertion, substitution, or a combination thereof, or replacement with a sequence having stronger activity, to further increase the activity of the expression regulatory region. The expression regulatory region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.
   Examples of the known stronger promoter may comprise cj1 to cj7 promoters (US Patent No. 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. 10584338 B2), O2 promoter (US Patent No. 10273491 B2), tkt promoter, yccA promoter, etc., but are not limited thereto.
3) The modification of a nucleotide sequence encoding an initiation codon or 5'-UTR region of the gene encoding the protein (polypeptide) may be, for example, replacing it with another initiation codon having a higher protein (polypeptide) expression rate than the endogenous initiation codon, or modifying it to encode a ribosome binding site (RBS) sequence having a higher protein (polypeptide) expression rate than the endogenous RBS sequence, but is not limited thereto.
4) and 5) The modification of the amino acid sequence or polynucleotide sequence of the protein (polypeptide) may be performed by introducing a mutation into the amino acid sequence of the protein (polypeptide) or the polynucleotide sequence encoding the protein (polypeptide) by deletion, insertion, substitution, or a combination thereof to increase the activity of the protein (polypeptide), or by replacing it with an amino acid sequence or a polynucleotide sequence which is modified to increase the activity, but is not limited thereto. The replacement may be performed by, for example, inserting the polynucleotide into the chromosome by homologous recombination, but is not limited thereto.
6) The introduction of a foreign polynucleotide exhibiting the activity of the protein (polypeptide) may be introduction, into a host cell (microorganism), of a foreign polynucleotide encoding a protein (polypeptide) exhibiting activity identical or similar to that of the protein (polypeptide). There is no limitation on its origin or sequence as long as the foreign polynucleotide exhibits activity identical or similar to that of the protein (polypeptide). The method used in the introduction may be performed by appropriately selecting a known transformation method by those skilled in the art. As the introduced polynucleotide is expressed in the host cell, the protein (polypeptide) may be produced and the activity thereof may be increased.
7) The codon optimization of the polynucleotide encoding the protein (polypeptide) may be codon optimization of an endogenous polynucleotide so as to enhance transcription or translation in a host cell (microorganism), or codon optimization of a foreign polynucleotide so as to perform optimized transcription and translation in a host cell (microorganism).
8) The analysis of the tertiary structure of the protein (polypeptide) to select and modify or chemically modify the exposed site may be, for example, to determine a template protein candidate according to the degree of similarity of the sequence by comparing the sequence information of a protein (polypeptide) to be analyzed with a database storing the sequence information of known proteins, to confirm the structure based on this, and to select and modify or chemically modify the exposed site to be modified or chemically modified.
9) The control of intracellular localization of the protein (polypeptide) may be targeting the protein (polypeptide) to a specific organelle within cells or to a specific intracellular space. For example, it may be targeting to the periplasm or cytoplasm through the addition or removal of a leader sequence that functions in targeting the protein (polypeptide), but is not limited thereto.

Such an increase of the activity of the protein (polypeptide) may be an increase of the activity or concentration of the corresponding protein (polypeptide), based on the activity or concentration of the protein (polypeptide) expressed in a wild-type or a host cell (microorganism) before modification, or an increase in the amount of a product resulting from the activity of the corresponding protein (polypeptide), but is not limited thereto.

The modification of a part or the entirety of the polynucleotide in the microorganism of the present disclosure may be induced by: (a) homologous recombination using a vector for chromosomal insertion into the microorganism or genome editing using engineered nuclease (e.g., CRISPR-Cas9), and/or (b) the treatment with light, such as ultraviolet light and radiation, and/or chemicals, but is not limited thereto.

The microorganism of the present disclosure may have an improved ability to produce an L-amino acid or a derivative thereof.

As used herein, the "L-amino acid or derivative thereof" comprises all L-amino acids or derivatives thereof that may be produced by microorganisms through metabolic processes from various carbon sources, and specifically, may comprise, but is not limited to, L-threonine, L-arginine, and L-homoserine.

In one specific embodiment of the present disclosure, the microorganism of the genus *Corynebacterium* may have an increased ability to produce any one or more L-amino acids or derivatives thereof selected from L-threonine, L-arginine, and L-homoserine, as compared to an unmodified microorganism.

Still another aspect of the present disclosure provides a method of producing an L-amino acid or a derivative thereof, the method comprising the step of culturing, in a medium, the microorganism comprising any one or more selected from the variant polypeptide of the present disclosure; the polynucleotide encoding the variant polypeptide; and the vector comprising the polynucleotide.

The microorganism is as described in other aspects.

As used herein, the term "culturing" means that the microorganism of the present disclosure is grown under appropriately controlled environmental conditions. In the present disclosure, the culturing process may be performed according to appropriate media or culture conditions known in the art. Such a culturing process may be easily adjusted for use by those skilled in the art according to the strain to be selected. Specifically, the culturing may be of a batch type, a continuous type, and/or a fed-batch type, but is not limited thereto.

The microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or decomposition products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner, but are not limited thereto.

The pH of the medium may be adjusted by adding compounds, such as ammonium hydroxide, potassium hydroxide, ammonia, phosphoric acid, sulfuric acid, etc., to the medium in an appropriate manner during culturing of the microorganism of the present disclosure. In addition, an anti-foaming agent, such as a fatty acid polyglycol ester, may be used to suppress foam formation during culturing. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or non-aerobic state, but this is not limited thereto.

Further, the culture medium may comprise metal salts, such as magnesium sulfate or iron sulfate, necessary for growth. Finally, in addition to the above substances, essential growth substances, such as amino acids and vitamins, may be used. Appropriate precursors may also be used in the culture medium. The above-mentioned raw materials may be added to the culture in a batch or continuous type in an appropriate manner during the culture process, but are not limited thereto.

In the present disclosure, the pH of the culture may be adjusted by adding compounds, such as ammonium hydroxide, potassium hydroxide, ammonia, phosphoric acid, sulfuric acid, etc., to the culture in an appropriate manner during culturing of the microorganism. In addition, an anti-foaming agent, such as a fatty acid polyglycol ester, may be used to suppress foam formation during culturing. In addition, oxygen or oxygen-comprising gas may be injected into the culture to maintain the aerobic state of the culture, or no gas may be injected, or nitrogen, hydrogen, or carbon dioxide gas may be injected to maintain the anaerobic or non-aerobic state, but this is not limited thereto.

In the culturing of the present disclosure, the culture temperature may be maintained at 20°C to 35°C, specifically 25°C to 35°C, and the culturing may be continued until the desired amount of a useful material is obtained, and may be carried out for about 10 hours to about 160 hours, about 20 hours to about 130 hours, about 24 hours to about 120 hours, about 36 hours to about 120 hours, about 48 hours to about 120 hours, about 48 hours or more, or about 48 hours, about 72 hours, or about 120 hours, but is not limited thereto.

The L-amino acid or derivative thereof produced by culturing of the present disclosure may be released into the medium or may remain in the cells.

In one specific embodiment of the present disclosure, the L-amino acid or derivative thereof may be any one or more selected from L-threonine, L-arginine, and L-homoserine, but is not limited thereto.

In one specific embodiment, the method of producing the L-amino acid or derivative thereof of the present disclosure may further comprise the steps of preparing the microorganism of the present disclosure, preparing a medium for culturing the strain, or a combination thereof (regardless of the order, in any order), for example, before the culturing step.

The method of producing the L-amino acid or derivative thereof of the present disclosure may further comprise the step of recovering the target material, specifically, the L-amino acid or derivative thereof from the cultured microorganism, a culture of the microorganism, a fermentation product of the microorganism, or the culture medium. The recovering step may be further comprised after the culturing step.

The recovering is to collect the desired L-amino acid or derivative thereof using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, etc., HPLC, and a combination thereof may be used, and the desired substance, specifically, the L-amino acid or derivative thereof may be recovered from the medium or the microorganism using suitable methods known in the art.

In addition, the method of producing the L-amino acid or derivative thereof of the present disclosure may further comprise a purification step. The purification may be performed by using an appropriate method known in the art. In an exemplary embodiment, when the method of producing the L-amino acid or derivative thereof of the present disclosure comprises both the recovering step and the purification step, the recovering step and the purification step may be performed continuously or discontinuously regardless of the order, or may be performed simultaneously or integrated into one step, but this is not limited thereto.

With regard to the method of the present disclosure, the variant polypeptide, introduction, and L-amino acid or derivative thereof, etc. are as described in other aspects.

Still another aspect of the present disclosure provides a composition for producing the L-amino acid or derivative thereof, the composition comprising any one or more selected from the variant polypeptide of the present disclosure; the polynucleotide encoding the variant polypeptide; the vector comprising the same; the microorganism comprising the vector; a culture of the microorganism; and a fermentation product of the microorganism.

The composition of the present disclosure may further comprise any appropriate excipient that is usually used in a composition for producing L-amino acids or derivatives thereof, and such excipients may comprise, for example, a preserving agent, a wetting agent, a dispersing agent, a suspending agent, a buffering agent, a stabilizing agent, an isotonic agent, etc., but are not limited thereto.

In one specific embodiment, each component present in the composition of the present disclosure may be comprised in a microbiologically effective amount, or in an amount that may be appropriately present in the composition for production.

With regard to the composition of the present disclosure, the variant polypeptide, introduction, and L-amino acid or derivative thereof, etc. are as described in other aspects.

Still another aspect of the present disclosure provides use of the microorganism having the improved ability to produce the L-amino acid or derivative thereof, into which any one or more selected from the variant polypeptide of the present disclosure; and the polynucleotide encoding the variant polypeptide are introduced, in producing the L-amino acid or derivative thereof.

With regard to the use of the present disclosure, the variant polypeptide, introduction, and L-amino acid or derivative thereof, etc. are as described in other aspects.

Still another aspect of the present disclosure provides a method of preparing the microorganism having the improved ability to produce the L-amino acid or derivative thereof, the method comprising the step of introducing any one or more selected from the variant polypeptide of the present disclosure; and the polynucleotide encoding the variant polypeptide into the microorganism of the genus *Corynebacterium* having the ability to produce the L-amino acid or derivative thereof.

Still another aspect of the present disclosure provides a method of increasing the ability to produce the L-amino acid or derivative thereof, the method comprising the step of introducing any one or more selected from the variant polypeptide of the present disclosure; and the polynucleotide encoding the variant polypeptide into the microorganism of the genus *Corynebacterium* having the ability to produce the L-amino acid or derivative thereof.

With regard to the method of the present disclosure, the variant polypeptide, introduction, and L-amino acid or derivative thereof, etc. are as described in other aspects.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Construction of Recombinant Vector for Introducing Heterologous Nicotinamide Nucleotide Transhydrogenase (pntAB)

### Example 1-1: Construction of Plasmid for Gene Insertion

In order to insert a heterologous nicotinamide nucleotide transhydrogenase (hereinafter, referred to as pntAB) gene into the chromosome of *Corynebacterium glutamicum*, NCgl1287, which is known as a transposon-encoding gene, was used as an insertion site (J Bacteriol. 2011 Mar; 193(5): 1237-1249). To replace the NCgl1287 gene with a heterologous pntAB, a vector for NCgl1287 deletion and target gene insertion was constructed. To construct the vector, PCR was performed using primer pairs SEQ ID NO: 1 and SEQ ID NO: 2, and SEQ ID NO: 3 and SEQ ID NO: 4, respectively, using the respective chromosomal DNA of *Corynebacterium glutamicum* ATCC13032 or ATCC13869 as templates. The primer sequences used to perform each PCR are shown in Table 1 below.

PfuUltra^{™} high-fidelity DNA polymerase (Stratagene) was used as a polymerase for the PCR reaction, and the PCR conditions were denaturation at 95°C for 30 seconds; denaturation at 55°C for 30 seconds; and polymerization at 72°C for 1 minute. The denaturation, annealing, and polymerization of the conditions were repeated for 28 cycles. As a result, DNA fragments of 720 bp and 854 bp were obtained, respectively. The obtained DNA products were purified using a PCR purification kit (QUIAGEN), respectively. The purified amplification product was treated with the restriction enzyme smal, and then cloned into a pDC24 vector (SEQ ID NO: 112) heat-treated at 65°C for 20 minutes using an infusion cloning kit (TaKaRa) according to the provided manual, thereby constructing pDC24△N1287 (13032) and pDC24△N1287 (13869) which are vectors for NCgl1287 deletion and target gene insertion.

**[Table 1]**

| SEQ ID NO. | Name | Sequence |
|---|---|---|
| 1 | Primer | AATTCGAGCTCGGTACCCAATGGAGCTGAAAGAAT |
| 2 | Primer | CTTCCTGATAGTCCCGGGACATTGTTTTTC |
| 3 | Primer | GAAAAACAATGTCCCGGGACTATCAGGAAG |
| 4 | Primer | GTCGACTCTAGAGGATCCCCATAAAAACAGGCAGAGG A |

### Example 1-2: Construction of Plasmid for pntAB Introduction

To further enhance the activity of pntAB to be introduced, a plasmid was constructed, in which an endogenous promoter of the pntAB gene to be introduced was replaced with PgapA.

First, a PgapA promoter fragment was obtained by PCR using chromosomal DNA of *Corynebacterium glutamicum* 13032 as a template and a primer pair of SEQ ID NO: 5 and SEQ ID NO: 6. Furthermore, an amino acid sequence of PntAB derived from *Corynebacterium variabile* and a nucleotide sequence encoding the same were obtained from the National Institutes of Health GenBank (NIH GenBank). Based on these sequences, the pntAB gene derived from *Corynebacterium variabile* was synthesized using Cosmogenetech's gene synthesis service. The synthesized pntAB gene as a template and primers of SEQ ID NO: 7 and SEQ ID NO: 8 were used to perform PCR. The primer sequences used to perform each of the above PCRs are shown in Table 2 below.

**[Table 2]**

| SEQ ID NO. | Name | Sequence |
|---|---|---|
| 5 | Primer | CCTGAAAAACAATGTCCCAACGACCGAGCCTATTG |
| 6 | Primer | GTTGTGTCTCCTCTAAAGAT |
| 7 | Primer | TCTTTAGAGGAGACACAACATGCTTATTGGTATTCCG AG |
| 8 | Primer | AACCAACTTCCTGATAGTCCCCTACAGGTGGGAGAT GATCT |

PfuUltra^{™} high-fidelity DNA polymerase (Stratagene) was used as a polymerase for the PCR reaction, and the PCR conditions were denaturation at 95°C for 30 seconds; denaturation at 55°C for 30 seconds; and polymerization at 72°C for 1 minute. The denaturation, annealing, and polymerization of the conditions were repeated for 28 cycles. As a result, a 472-bp DNA fragment of the PgapA promoter region and a 3041-bp DNA fragment of the pntAB gene of *Corynebacterium variabile* were obtained, respectively.

The amplified promoter and the pntAB DNA fragment of *Corynebacterium variabile* as templates and the primer pair of SEQ ID NO: 5 and SEQ ID NO: 8 were used to perform PCR. PCR was performed under the following conditions: denaturation at 95°C for 5 minutes, followed by 28 cycles of denaturation at 95°C for 30 seconds, annealing at 55°C for 30 seconds, and polymerization at 72°C for 2 minutes, and then polymerization at 72°C for 5 minutes.

As a result, about 3.5 kb of pntAB DNA fragment encoding nicotinamide nucleotide transhydrogenase of *Corynebacterium variabile* which was linked to the PgapA promoter was amplified.

The amplified product was purified using a PCR purification kit (QUIAGEN) and used as an insert DNA fragment for vector construction. The purified amplification product and the pDC24△N1287(13032) or pDC24△N1287(13869) vector, which had been heat-treated at 65°C for 20 minutes after treatment with the restriction enzyme smal, were cloned in a molar concentration (M) ratio of 2:1 using an infusion cloning kit (TaKaRa) according to the provided manual, thereby constructing pDC24△N1287(13032)::PgapA_pntAB and pDC24△N1287(13869)::PgapA_pntAB vectors for introducing the pntAB gene of *Corynebacterium variabile* onto the chromosome of *Corynebacterium glutamicum.*

### Example 2: Construction of Plasmids for pntAB Mutation Introduction

PCR was performed using pDC24△N1287(13032)::PgapA_pntAB constructed in Example 1-2 as a template and primer pairs of Table 3. PCR was performed under the following conditions: denaturation at 95°C for 30 seconds, followed by 25 cycles of denaturation at 95°C for 30 seconds, annealing at 55°C for 30 seconds, polymerization at 68°C for 5 minutes, and then polymerization at 68°C for 3 minutes.

The obtained DNA products were purified using a PCR purification kit (QUIAGEN), thereby preparing various vectors for pntAB mutation introduction, as listed in Table 3.

**[Table 3]**

| Plasmid for pntAB mutation | SEQ ID NO. | Sequence name | Sequence (5'-> 3') |
|---|---|---|---|
| pDC24ΔAN1287( 13032)::PgapA_ pntA(T458A)B | 14 | pDC24ΔN1287 ::PgapA_ pntA(T458A)B F | GCAAATGCTATTTCTGGCATCA TTTTGG |
| | 15 | pDC24ΔN1287 ::PgapA_ pntA(T458A)B R | CCAGAAATAGCATTTGCTTCGG ACATGAGTGGGG |
| pDC24ΔN1287( 13032)::PgapA_ pntAB(N55T) | 16 | pDC24ΔN1287::PgapA_ pntAB(N55T) F | ACCACCTTCGGTATCGCTGGT ATG |
| | 17 | pDC24ΔN1287::PgapA_ pntAB(N55T) R | CAGCGATACCGAAGGTGGTGC CGCGCGC |
| pDC24ΔAN1287( 13032)::PgapA_ pntAB(N198T) | 18 | pDC24ΔN1287::PgapA_ pntAB(N198T) F | ACCCTCGCAATCATTGTGTTGT CCG |
| | 19 | pDC24ΔN1287::PgapA_ pntAB(N198T) R | ACACAATGATTGCGAGGGTGA GCAGGTTACGGCCG |
| pDC242ΔN1287 (13032)::PgapA _pntAB(S289A) | 20 | pDC24ΔN1287::PgapA_ pntAB(S289A) F | GCATCTGGTGCTTACTTGTCCT ATATC |
| | 21 | pDC24ΔN1287::PgapA_ pntAB(S289A) R | AAGTAAGCACCAGATGCACCG ACGAGGGCG |
| pDC24ΔAN1287( 13032)::PgapA_ pntAB(Y296F) | 22 | pDC24ΔN1287::PgapA_ pntAB(Y296F) F | TTCATCATGTGTAAGGCTATGA ACCG |
| | 23 | pDC24ΔN1287::PgapA_ pntAB(Y296F) R | TAGCCTTACACATGATGAAGGA CAAGTAAGCACCAGAG |
| pDC24ΔN1287( 13032)::PgapA_ pntAB(T431C) | 24 | pDC24ΔN1287::PgapA_ pntAB(T431C) F | TGCGTGAATCCAATCGCTGAA GAGC |
| | 25 | pDC24ΔN1287::PgapA_ pntAB(T431C) R | AGCGATTGGATTCACGCAATC GTTGGCGCCAATAAC |
| pDC24ΔN1287( 13032)::PgapA_ | 26 | pDC24ΔN1287::PgapA_ pntAB(I435C) F | TGCGCTGAAGAGCCAGGTTCT CC |
| pntAB(I435C) | 27 | pDC24ΔN1287::PgapA_ pntAB(I435C) R | CCTGGCTCTTCAGCGCATGGA TTCACGGTATCGTTGG |
| pDC24ΔN1287( 13032)::PgapA_ pntAB(I443C) | 28 | pDC24ΔN1287::PgapA_ pntAB(I443C) F | TGCGCGGGTATGCCAGTCTTG |
| | 29 | pDC24ΔN1287::PgapA_ pntAB(I443C) R | GACTGGCATACCCGCGCAAGG AGAACCTGGCTCTTC |
| pDC24ΔN1287( 13032)::PgapA_ pntAB(M446C) | 30 | pDC24ΔN1287::PgapA_ pntAB(M446C) F | TGCCCAGTCTTGAAGGTTTGG GACG |
| | 31 | pDC24ΔN1287::PgapA_ pntAB(M446C) R | AACCTTCAAGACTGGGCAACC CGCAATAGGAGAACCTG |
| pDC24ΔN1287( 13032)::PgapA_ pntAB(G467C) | 32 | pDC24ΔN1287::PgapA_ pntAB(G467C) F | TGCTACGCTGGCGTTCAAAAC C |
| | 33 | pDC24ΔN1287::PgapA_ pntAB(G467C) R | TTGAACGCCAGCGTAGCAGGC GCCCATAGAGCG |
| pDC24ΔAN1287( 13032)::PgapA_ pntAB(Y468C) | 34 | pDC24ΔN1287::PgapA_ pntAB(Y468C) F | TGCGCTGGCGTTCAAAACCC |
| | 35 | pDC24ΔN1287::PgapA_ pntAB(Y468C) R | GGTTTTGAACGCCAGCGCAGC CGGCGCCCATAG |

### Example 3: Preparation of pntAB Mutation-Introduced L-Amino Acid Strain and Evaluation of Amino Acid-Producing Ability

### Example 3-1: Preparation of pntAB Mutation-Introduced L-Threonine-Producing Strain and Evaluation of Threonine-Producing Ability

The vectors for introducing pntAB mutations which were constructed in Example 2-2 were each transformed into a threonine-producing strain, and threonine productions were analyzed after culture. In detail, each constructed vector was transformed into the threonine-producing strain *Corynebacterium glutamicum* KCCM 12502P (CA09-0903, Korean Patent No. 10-2126951) by electroporation to prepare strains expressing the pntAB mutations, which were cultured as follows.

First, each strain was inoculated into a 250-ml corner-baffled flask comprising 25 ml of a seed medium and cultured at 30°C for 20 hours with shaking at 200 rpm. Next, 1 ml of the seed culture was inoculated into a 250-ml corner-baffled flask comprising 24 ml of a production medium and cultured at 32°C for 24 hours with shaking at 200 rpm.

### <Seed Medium (pH 7.0)>

20 g of Glucose, 10 g of Peptone, 5 g of Yeast Extract, 1.5 g of Urea, 4 g of KH₂PO₄, 8 g of K₂HPO₄, 0.5 g of MgSO₄ 7H₂O, 100 µg of Biotin, 1000 µg of Thiamine HCl, 2000 µg of Calcium Pantothenate, 2000 µg of Nicotinamide (based on 1 liter of distilled water)

### <Production Medium (pH 7.2)>

30 g of Glucose, 2 g of KH₂PO₄, 3 g of Urea, 40 g of (NH₄)₂SO₄, 2.5 g of Peptone, 5 g (10 ml) of CSL (Sigma), 0.5 g of MgSO₄•7H₂O, 400 mg of Leucine, 20 g of CaCO₃ (based on 1 liter of distilled water)

After completing the culture, L-threonine production amounts were measured using liquid high-performance chromatography (HPLC), and the concentrations are shown in Table 4 below.

**[Table 4]**

| Threonine-producing ability of microorganism expressing PntAB variant | | |
|---|---|---|
| Name of strain | L-threonine concentration (g/L) | Percentage (%) of concentration, relative to wild-type pntAB strain |
| CA09-0903 ΔN1287::PgapA_pntAB | 5.6 | 100 |
| CA09-0903 ΔN1287::PgapA_pntA(T458A)B | 5.0 | 89 |
| CA09-0903 ΔN1287::PgapA_pntAB(N55T) | 2.8 | 50 |
| CA09-0903 ΔN1287::PgapA_pntAB(N198T) | 5.5 | 98 |
| CA09-0903 ΔN1287::PgapA_pntAB(S289A) | 5.9 | 105 |
| CA09-0903 ΔN1287::PgapA_pntAB(Y296F) | 5.4 | 96 |
| CA09-0903 ΔN1287::PgapA_pntAB(T431C) | 3.2 | 57 |
| CA09-0903 ΔN1287::PgapA_pntAB(I435C) | 5.6 | 100 |
| CA09-0903 ΔN1287::PgapA_pntAB(I443C) | 3.3 | 59 |
| CA09-0903 ΔN1287::PgapA_pntAB(M446C ) | 2.3 | 41 |
| CA09-0903 ΔN1287::PgapA_pntAB(G467C ) | 2.1 | 38 |
| CA09-0903 ΔN1287::PgapA_pntAB(Y468C) | 3.4 | 61 |

As a result, as shown in Table 4, the parent strain, *Corynebacterium glutamicum* CA09-0903 △N1287::PgapA_pntAB, was confirmed to produce about 5.6 g/L of threonine. Among the strains expressing pntAB variants, CA09-0903 △N1287::PgapA_pntAB(S289A) which is the strain expressing pntAB (S289A) showed a 5% increase in threonine production, as compared to the wild-type pntAB (C.va) strain.

### Example 3-2: Preparation of pntAB(S289A) Mutation-Introduced L-Arginine-Producing Strain and Evaluation of Production Ability

The pntAB(S289A) mutation, which demonstrated superior production ability in Example 3-1, was introduced into an arginine-producing strain to determine whether arginine production was enhanced. In detail, similar to the method described in Example 2, PCR was performed using the pDC24△N1287(13869)::PgapA_pntAB vector as a template and a primer pair of SEQ ID NO: 20 and SEQ ID NO: 21 to construct a pDC24△N1287(13869)::PgapA_pntAB(S289A) vector. This vector was then transformed into the arginine-producing strain *Corynebacterium glutamicum* CJR2 (Korean Patent Application No. 10-2023-0048232) by electroporation.

To determine the arginine-producing ability of the strain, the strain was cultured by the following method.

First, the strain was inoculated into a 250-ml corner-baffled flask comprising 25 ml of a seed medium and cultured at 30°C for 20 hours with shaking at 200 rpm. Next, 1 ml of the seed culture was inoculated into a 250-ml corner-baffled flask comprising 24 ml of a production medium and cultured at 30°C for 72 hours with shaking at 200 rpm.

### <Seed Medium (pH 7.0)>

20 g of Glucose, 10 g of Peptone, 5 g of Yeast Extract, 1.5 g of Urea, 4 g of KH₂PO₄, 8 g of K₂HPO₄, 0.5 g of MgSO₄ 7H₂O, 100 µg of Biotin, 1000 µg of Thiamine HCl, 2000 µg of Calcium Pantothenate, 2000 µg of Nicotinamide (based on 1 liter of distilled water)

### <Production Medium (pH 7.0)>

6% Glucose, 3% Ammonium Sulfate, 0.1% Potassium Phosphate Monobasic, 0.2% Magnesium Sulfate Heptahydrate, 1.5% Corn Steep Liquor, 1% NaCl, 0.5% Yeast Extract, 100 mg/L Biotin

After completing the culture, L-arginine production amount was measured using liquid high-performance chromatography (HPLC), and the concentration is shown in Table 5 below.

**[Table 5]**

| Arginine-producing ability of microorganism expressing PntAB(S289A) variant | | |
|---|---|---|
| Name of strain | L-arginine concentration (g/L) | Percentage (%) of concentration, relative to wild-type pntAB strain |
| CJR2 ΔN1287::PgapA_pnt AB | 2.4 | 100 |
| CJR2ΔN287::Pgap A_pntAB(S289A) | 2.6 | 108 |

As a result, as shown in Table 5, the mutation-introduced CJR2△N1287::PgapA_pntAB(S289A) strain showed an 8% increase in arginine production, as compared to the CJR2 △N1287::PgapA_pntAB strain.

### Example 3-3: Preparation of pntAB(S289A) Mutation-Introduced L-Homoserine-Producing Strain and Evaluation of Production Ability

The pntAB(S289A) mutation, which demonstrated superior production ability in Example 3-1, was introduced into a homoserine-producing strain. In detail, the pDC24△N1287(13032)::PgapA_pntAB vector constructed in Example 2 was transformed into *Corynebacterium glutamicum* KCCM12120P (Korean Patent No. 10-1947959) to prepare a strain expressing pntAB(S289A), which was then cultured by the following method.

In detail, the strain was inoculated into a 250-ml corner-baffled flask comprising 25 ml of a production medium and cultured at 30°C for 24 hours with shaking at 200 rpm.

### <Production Medium (pH 7.0)>

45 g of Glucose, 20 g of (NH₄)₂SO₄, 1.2 g of MgSO₄·7H₂O, 1.1 g of KH₂PO₄, 900 µg of Biotin, 4500 µg of Thiamine Hydrochloride, 4500 µg of Calcium-Pantothenic acid, 30 g of CaCO₃ (based on 1 liter of distilled water)

After completing the culture, L-homoserine production amount was measured using liquid high-performance chromatography (HPLC), and the concentration is shown in Table 6 below.

**[Table 6]**

| Homoserine-producing ability of microorganism expressing PntAB(S289A) variant | | |
|---|---|---|
| Name of strain | L-homoserine concentration (g/L) | Percentage (%) of concentration, relative to parent strain |
| KCCM12120P ΔN1287(13032)::PgapA_pntAB | 0.56 | 100 |
| KCCM12120P ΔN1287(13032)::PgapA_pntAB(S289 A) | 0.62 | 110 |

As a result, as shown in Table 6, the mutation-introduced KCCM12120P △N1287(13032)::PgapA_pntAB(S289A) strain showed a 10% increase in homoserine production, as compared to the KCCM12120P △N1287(13032)::PgapA_pntAB strain.

### Example 4: Introduction of Saturated Mutagenesis into Amino acid at Position 289 of PntB and Comparison of Threonine-Producing ability in Strains Expressing The Same

### Example 4-1: Preparation of Vector and Strain for Inserting Variant Amino Acid Substitution

To examine whether expression of the pntAB(S289A) variant showed the effect of increasing the amino acid yield, as compared to the wild-type strain, serine which is an amino acid at position 289 of pntB was mutated to other amino acids to investigate the effectiveness of the position 289 on improving L-threonine production.

Accordingly, to construct vectors expressing variants in which serine at position 289 of pntB was substituted with 19 amino acids other than serine, site-directed mutagenesis was performed using pDC24△N1287(13032)::PgapA_pntAB, constructed in Example 1, as a template, by the following method.

**[Table 7]**

| Composition of PCR reaction for site-directed mutagenesis | |
|---|---|
| Composition | Content (µl) |
| 10X pfu-X Buffer | 5 |
| 10 mM dNTP Mix | 1 |
| pfu-X Polymerase | 1 |
| Mutagenic forward primer (5 pmol) | 2 |
| Mutagenic reverse primer (5 pmol) | 2 |
| template DNA, 200 ng/µl | 1 |
| dH₂O | 38 |
| Total | 50 |

**[Table 8]**

| PCR conditions for site-directed mutagenesis | | |
|---|---|---|
| Number of cycles | Temperature | Time |
| 1 cycle | 95°C | 5 min |
| 18 cycles | 95°C | 30 sec |
| | 60°C | 1 min |
| | 68°C | 10 min |

In detail, pDC24△N1287(13032)::PgapA_pntAB was used as a template, and each primer pair in Table 9 was added to prepare a PCR composition as in Table 7, and PCR was performed under the conditions as in Table 8. After PCR was completed, 1 µl of Dpnl restriction enzyme was added and treated at 37°C for 1 hour. 3 µl of Dpnl-treated DNA was transformed into DH5a competent cells to obtain variant plasmids of pDC24△N1287(13032)::PgapA_pntAB as described in Table 9.

**[Table 9]**

| Primer set for mutagenesis for constructing variant pntAB plasmid | | |
|---|---|---|
| Variant pntAB plasmid | SEQ ID NO. | Nucleotide sequence |
| pDC24ΔN1287(13032)-PgapA_pntAB S289A | SEQ ID NO: 36 | CCTCGTCGGTGCATCTGGTGCTTA CT |
| | SEQ ID NO: 37 | ACAAGTAAGCACCAGATGCACCGA CGA |
| pDC24ΔN1287(13032)-PgapA_pntAB S289C | SEQ ID NO: 38 | CCTCGTCGGTTGCTCTGGTGCTTA CT |
| | SEQ ID NO: 39 | ACAAGTAAGCACCAGAGCAACCG ACGA |
| pDC24ΔN1287(13032)-PgapA_pntAB S289D | SEQ ID NO: 40 | CCTCGTCGGTGATTCTGGTGCTTA CT |
| | SEQ ID NO: 41 | ACAAGTAAGCACCAGAATCACCGA CGA |
| pDC24ΔN1287(13032)-PgapA_pntAB S289E | SEQ ID NO: 42 | CCTCGTCGGTGAATCTGGTGCTTA CT |
| | SEQ ID NO: 43 | ACAAGTAAGCACCAGATTCACCGA CGA |
| pDC24ΔN1287(13032)-PgapA_pntAB S289F | SEQ ID NO: 44 | CCTCGTCGGTTTCTCTGGTGCTTA CT |
| | SEQ ID NO: 45 | ACAAGTAAGCACCAGAGAAACCGA CGA |
| pDC24ΔN1287(13032)-PgapA_pntAB S289G | SEQ ID NO: 46 | CCTCGTCGGTGGCTCTGGTGCTTA CT |
| | SEQ ID NO: 47 | ACAAGTAAGCACCAGAGCCACCG ACGA |
| pDC24ΔN1287(13032)-PgapA_pntAB S289H | SEQ ID NO: 48 | CCTCGTCGGTCACTCTGGTGCTTA CT |
| | SEQ ID NO: 49 | ACAAGTAAGCACCAGAGTGACCG ACGA |
| pDC24ΔN1287(13032)P gapA_pntAB S289I | SEQ ID NO: 50 | CCTCGTCGGTATCTCTGGTGCTTA CT |
| | SEQ ID NO: 51 | ACAAGTAAGCACCAGAGATACCGA CGA |
| pDC24ΔN1287(13032)-PgapA_pntAB S289K | SEQ ID NO: 52 | CCTCGTCGGTAAGTCTGGTGCTTA CT |
| | SEQ ID NO: 53 | ACAAGTAAGCACCAGACTTACCGA CGA |
| pDC24ΔN1287(13032)-PgapA_pntAB S289L | SEQ ID NO: 54 | CCTCGTCGGTCTTTCTGGTGCTTA CT |
| | SEQ ID NO: 55 | ACAAGTAAGCACCAGAAAGACCGA CGA |
| pDC24ΔN1287(13032)-PgapA_pntAB S289M | SEQ ID NO: 56 | CCTCGTCGGTATGTCTGGTGCTTA CT |
| | SEQ ID NO: 57 | ACAAGTAAGCACCAGACATACCGA CGA |
| pDC24ΔN1287(13032)-PgapA_pntAB S289N | SEQ ID NO: 58 | CCTCGTCGGTAATTCTGGTGCTTA CT |
| | SEQ ID NO: 59 | ACAAGTAAGCACCAGAATTACCGA CGA |
| pDC24β-PgapA_pntAB S289P | SEQ ID NO: 60 | CCTCGTCGGTCCCTCTGGTGCTTA CT |
| | SEQ ID NO: 61 | ACAAGTAAGCACCAGAGGGACCG ACGA |
| pDC24ΔN1287(13032)-PgapA_pntAB S289Q | SEQ ID NO: 62 | CCTCGTCGGTCAATCTGGTGCTTA CT |
| | SEQ ID NO: 63 | ACAAGTAAGCACCAGATTGACCGA CGA |
| pDC24ΔN1287(13032)- | SEQ ID NO: 64 | CCTCGTCGGTCGTTCTGGTGCTTA CT |
| PgapA_pntAB S289R | SEQ ID NO: 65 | ACAAGTAAGCACCAGAACGACCG ACGA |
| pDC24ΔN1287(13032)-PgapA_pntAB S289T | SEQ ID NO: 66 | CCTCGTCGGTACCTCTGGTGCTTA CT |
| | SEQ ID NO: 67 | ACAAGTAAGCACCAGAGGTACCGA CGA |
| pDC24ΔN1287(13032)-PgapA_pntAB S289V | SEQ ID NO: 68 | CCTCGTCGGTGTGTCTGGTGCTTA CT |
| | SEQ ID NO: 69 | ACAAGTAAGCACCAGACACACCGA CGA |
| pDC24ΔN1287(13032)-PgapA_pntAB S289W | SEQ ID NO: 70 | CCTCGTCGGTTGGTCTGGTGCTTA CT |
| | SEQ ID NO: 71 | ACAAGTAAGCACCAGACCAACCGA CGA |
| pDC24ΔN1287(13032)-PgapA_pntAB S289Y | SEQ ID NO: 72 | CCTCGTCGGTTACTCTGGTGCTTA CT |
| | SEQ ID NO: 73 | ACAAGTAAGCACCAGAGTAACCGA CGA |

The constructed vectors were each transformed into a threonine-producing strain (CA09-0903) by electroporation according to the method of Example 3, and through a second crossover process, 19 types of strains were obtained, in which the variant pntAB gene was inserted on the chromosome.

### Example 4-2: Evaluation of Threonine-Producing Ability of pntAB Mutant Strains

The 19 types of strains prepared in Example 4-1 and the previously prepared CA09-0903 △N1287(13032)::PgapA_pntAB strain were cultured using the L-threonine production flask medium and culture method of Example 3-1. After completing the culture, L-threonine production amounts were measured by HPLC, and shown in Table 10.

**[Table 10]**

| L-threonine production of strain introduced with 289^{th} amino acid-mutated pntAB | | |
|---|---|---|
| Strain | Threonine production (g/L) | Percentage of concentration, relative to parent strain (%) |
| CA09-0903ΔN1287(13032)::P gapA_pntAB | 5.6 | 100 |
| CA09-0903ΔN1287(13032)::P gapA_pntAB(S289A) | **5.9** | 105 |
| CA09-0903ΔN1287(13032)::P gapA_pntAB(S289C) | **5.7** | 102 |
| CA09-0903ΔN1287(13032)::P gapA_pntAB(S289D) | 5.5 | 98 |
| CA09-0903ΔN1287(13032)::P gapA_pntAB(S289E) | 5.4 | 96 |
| CA09-0903ΔN1287(13032)::P gapA_pntAB(S289F) | 1.6 | 29 |
| CA09-0903ΔN1287(13032)::P gapA_pntAB(S289G) | 5.3 | 95 |
| CA09-0903ΔN1287(13032)::P gapA_pntAB(S289H) | 2.8 | 50 |
| CA09-0903ΔN1287(13032)::P gapA_pntAB(S289I) | **5.7** | 102 |
| CA09-0903ΔN1287(13032)::P gapA_pntAB(S289K) | 5.3 | 95 |
| CA09-0903ΔN1287(13032)::P gapA_pntAB(S289L) | 1.4 | 25 |
| CA09-0903ΔN1287(13032)::P gapA_pntAB(S289M) | **5.8** | 104 |
| CA09-0903ΔN1287(13032)::P gapA_pntAB(S289N) | 5.4 | 96 |
| CA09-0903ΔN1287(13032)::P gapA_pntAB(S289P) | 1.3 | 23 |
| CA09-0903ΔN1287(13032)::P gapA_pntAB(S289Q) | 1.2 | 21 |
| CA09-0903ΔN1287(13032)::P gapA_pntAB(S289R) | 1.4 | 25 |
| CA09-0903ΔN1287(13032)::P gapA_pntAB(S289T) | **5.8** | 104 |
| CA09-0903ΔN1287(13032)::P gapA_pntAB(S289V) | 1.1 | 20 |
| CA09-0903ΔN1287(13032)::P gapA_pntAB(S289W) | 1.2 | 21 |
| CA09-0903ΔN1287(13032)::P gapA_pntAB(S289Y) | 5.6 | 100 |

As shown in Table 10, the strains, into which each of the five types of variants (S289A, S289C, S289I, S289M, or S289T) was inserted among 19 types of variants at position 289 of pntB protein, showed improvement in the fermentation yields, as compared to the strain into which the wild-type pntAB was introduced. Among them, CA09-0903 △N1287(13032)::PgapA_pntAB(S289A), which is a strain expressing the pntAB (S289A) variant, showed the most effective threonine production of 5% increase, as compared to the wild-type pntAB strain.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A variant polypeptide of a nicotinamide nucleotide transhydrogenase beta subunit, wherein an amino acid corresponding to position 289 of SEQ ID NO: 11 is substituted with alanine; cysteine; isoleucine; methionine; or threonine.

2. The variant polypeptide of claim 1, wherein an amino acid sequence of the variant polypeptide has 90% or more identity to the amino acid sequence of SEQ ID NO: 11.

3. The variant polypeptide of claim 1, wherein the variant polypeptide consists of an amino acid sequence of SEQ ID NO: 74, SEQ ID NO: 76, SEQ ID NO: 78, SEQ ID NO: 80, or SEQ ID NO: 82.

4. A nicotinamide nucleotide transhydrogenase comprising the variant polypeptide of any one of claims 1 to 3.

5. A polynucleotide encoding the variant polypeptide of any one of claims 1 to 3.

6. A microorganism comprising any one or more selected from the variant polypeptide of any one of claims 1 to 3; and a polynucleotide encoding the variant polypeptide.

7. The microorganism of claim 6, wherein the microorganism is a microorganism of the genus *Corynebacterium.*

8. The microorganism of claim 7, wherein the microorganism of the genus *Corynebacterium* is *Corynebacterium glutamicum.*

9. The microorganism of claim 7, wherein the microorganism has an increased ability to produce an L-amino acid or a derivative thereof, as compared to a microorganism comprising the polypeptide of SEQ ID NO: 11 or a polynucleotide encoding the same.

10. The microorganism of claim 9, wherein the L-amino acid or derivative thereof is L-threonine, L-arginine, or L-homoserine.

11. A method of producing an L-amino acid or a derivative thereof, the method comprising the step of culturing the microorganism of claim 6 in a medium.

12. The method of claim 11, further comprising the step of recovering the L-amino acid or the derivative thereof from the cultured microorganism; a culture of the microorganism; a fermentation product of the microorganism; or the culture medium.

13. The method of claim 12, wherein the L-amino acid or derivative thereof is L-threonine, L-arginine, or L-homoserine.

14. Use of the variant polypeptide of any one of claims 1 to 3; or a microorganism comprising the variant polypeptide or a polynucleotide encoding the variant polypeptide in producing an L-amino acid or a derivative thereof.
